# EUROPEAN PATENT APPLICATION

(11) **EP 3 321 846 A1**
(43) Date of publication of application: **16.05.2018**
(21) Application number: 16198855.5
(22) Date of filing: 15.11.2016
(51) Int. Cl.: G06K 9/00, G06K 9/22

(54) **SYSTEMS AND METHODS FOR SECURE BIOMETRIC SAMPLE RAW DATA STORAGE**

(71) Applicant: Mastercard International Incorporated, New York, NY 10577 (US)
(72) Inventor: NOWAK, Dawid, Dublin, 15 (IE); DRUTA, Vladut, Dublin, 1 (IE)
(74) Representative: Murgitroyd & Company

(57) **Abstract**

A method for biometric template extraction and secure biometric sample raw data storage, the method comprising enrolling, at a terminal, at least one biometric sample of a user, storing, in a secure storage on the terminal, the raw data of the biometric sample, extracting, by an extraction application executed on the terminal, a biometric template from the raw data of the sample, fragmenting, by a raw sample splitter executed on the terminal, the raw data of the biometric sample, distributing the fragments of the raw data of the biometric samples from the terminal to independent entities, preferably via a secure communication channel, and deleting the biometric sample raw data from the secure storage of the terminal.

## Description

### Technical Field

The present disclosure relates to the field of biometric authentication and biometric sample raw data handling. More particularly, the present disclosure relates to methods and systems for distributed storage of biometric sample raw data. Moreover, the disclosure relates to a computer program product and a computer readable medium. The computer readable medium comprises computer-executable instructions, which, when executed by the respective devices being equipped with processors cause the devices to perform the method steps of the disclosure on the respective device interacting with the respective other device.

### Background

Biometric authentication has become a valuable and commonly used tool. This is, on the one hand, because of the comprehensive availability of suitable scanning devices, e.g. in form of smart phones providing scanning functionality on the other hand because of improved network connectivity. According to this, making use of existing hardware, biometric authentication methods can be implemented in many situations, such as authentication, identification and also authorization processes.

Similar to hand written signatures, which are normally compared by human beings with a provided proof of the signature, e.g. when paying with a credit card, also biometric scans need to be compared to a verified proof. Similar to providing a proof of a hand written signature on the backside of a credit card after receiving the card, when enrolling for biometric authentication, a master of the used biometric sample is taken. Different to the human based comparison of hand written signatures, in biometric authentication the captured biometric is not compared with the raw data of the biometric sample but with a specific template stored in a biometric database in order to verify the individual is the person it claims to be.

Accordingly, different to the handwritten signature (raw data) on the backside of a credit card, for biometric authentication, certain characterising features are extracted from the raw data sample. In subsequent biometric identification or authentication procedures making use of the biometric authentication, the comparison is based on the extracted features only. By doing this, the authentication process is significantly accelerated, as less data has to be processed, and, in cases of the biometric scanning device and the authentication device being connected over a network (e.g. a biometric authentication application installed on a smartphone communication of an authentication server), less data needs to be transferred.

In case of any doubt with respect to the validity of the biometric verification, the original raw data of the biometric sample might need to be used, such that the original raw data of the biometric sample has to be stored. However, as the original raw data of the biometric sample could also be used for the biometric authentication, as it matches with the template by definition, secure storage of the raw data is fundamental for security of the system. Even though possible, safe storage of the data in physical form, e.g. on a memory stick in a safe deposit box seems unpractical, especially when the original raw data is required for verification purposes. Accordingly, there is a need for secure biometric sample raw data storage.

### Summary of the invention

The present disclosure provides one or more solutions to the problems and disadvantages of the background art. Other technical advantages of the present disclosure will be readily apparent to one skilled in the art from the following description and claims.

The present disclosure is directed to a computer implemented method for biometric template extraction and secure biometric sample raw data storage, the method comprising:
- enrolling, at a terminal, at least one biometric sample of a user;
- storing, in a secure storage on the terminal, the raw data of the biometric sample;
- extracting, by an extraction application executed on the terminal, a biometric template from the raw data of the sample;
- fragmenting, by a raw sample splitter executed on the terminal, the raw data of the biometric sample;
- distributing the fragments of the raw data of the biometric samples from the terminal to independent entities, preferably via a secure communication channel; and
- deleting the biometric sample raw data from the secure storage of the terminal.

The present disclosure is further directed to a computer implemented method for biometric template extraction and secure biometric sample raw data storage, the method comprising:
- enrolling, at a terminal, at least one biometric sample of a user;
- transmitting, preferably via a secure communication channel, the raw data of the biometric sample from the terminal to a server;
- extracting, by an extraction application executed on the server, a biometric template from the raw data of the sample;
- fragmenting, by a raw sample splitter executed on the server, the raw data of the biometric sample;
- distributing the fragments of the raw data of the biometric samples from the server to independent entities, preferably via a secure communication channel; and
- deleting the biometric sample raw data from the memory of the server.

The present disclosure includes multiple aspects for secure biometric sample raw data storage. The biometric sample may be based on aspects of human physiology, chemistry or behaviour that show characteristics and as such can be used for biometric authentication. Non-limiting examples for characteristics to be used for biometric authentication may be any one, or combination, of the following: fingerprint, palm veins, face recognition, DNA, palm print, hand geometry, iris recognition, retina and odour/scent but also behavioural characteristics such as typing rhythm, gait, and voice. Raw data of the biometric data may be any form of unprocessed and/or uncompressed data of the captured information of the respective characteristic(s) to be used for biometric authentication. A terminal may be any device capable of capturing information related to the characteristic(s) used, such as, for example, smartphones, personal computers, tablet computers comprising detection means for the respective characteristic(s), such as cameras or scanning devices, microphones, and configured to execute a respective application, but also dedicated biometric scanning devices and combined devices consisting of detection and processing units. The terminal may also comprise communication means and may also be connected to a network, such as the internet.

When the user uses the biometric authentication system for the first time or when the user starts using the biometric authentication system, the enrolment process is initiated. The biometric authentication system may be based on or may be accessible through a dedicated application executed on the terminal or a plugin, an inFrame solution, a SDK or the like implemented on a web site.

During the enrolment, biometric information from the user is captured by the respective detection means and stored. Storage may be on the terminal and/or on a server, however, storage of the captured data (raw data of the biometric sample) is preferably on a secure storage medium. If the raw data of the biometric sample is transmitted from the terminal to a server a secure communication channel may be used.

After the biometric information has been captured, the resulting raw data of the biometric sample is further processed by an extraction application. The extraction application may be executed on the terminal or on the server and may be integrated and as such forming part of other applications of the biometric authentication system. The extraction application extracts characteristic features of the raw data of the biometric sample and thereby creates a biometric template. The features to be extracted depend on the biometric sample used and are known to the person skilled in the art. The biometric template contains at least some characterising features of the biometric sample which will than be used for biometric authentication. The amount of features may depend on the required security level and/or on the available processing capabilities. The template creation may be implemented in the extraction application or may be processed by an addition template creation application.

After feature extraction and template creation the biometric sample raw data is fragmented by a raw data splitter. The raw data splitter may be executed on the terminal or on the server. After fragmentation of the biometric sample raw data the fragments are distributed to certain, predefined entities for storage and the biometric sample raw data is deleted. The number of fragments depends on the number of target entities, e.g. if fragments of the biometric sample raw data are to be stored on three external entities and on the terminal, the number of fragments has to be 4. In general, the fragmenting process results in at least two fragments. Independent entities used for storage may be physically separated storage entities, electronically separated storage entities and/or distinct and separated legal entities. The physically and/or electronically separated storage entities may be held by the same and/or by different legal entities or combinations thereof. By way of distributing the fragments to different entities, the level of security may be increased.

Fragmenting of the biometric sample raw data may comprise fragmenting the data into equally sized fragments or into fragments of different sizes. If the biometric sample raw data is fragmented into fragments of different sizes, the respective sizes may be based on a predefined sizing pattern or, alternatively, based on a dynamically created random sizing pattern. The fragmenting may be based on the digital data, i.e. separating the file describing the data into portions or on the described data file, e.g. cutting an image of a fingerprint into 4 pieces. In case the recombination of the fragments to regain the initial raw data requires further information, respective reconstruction instructions may be stored as an individual file or may be added to at least one of the fragment files, e.g. as a meta tag. If the reconstruction instructions are stored in an individual file, this file may be stored together with at least one fragment or may be sent for storage to an additional entity. Alternatively, the fragmenting or reconstruction may be defined as preferences and as such may be stored in a configuration file on the server or on the terminal.

In case of verification of a biometric authentication process being required, the fragments of the biometric sample raw data can be consolidated and the biometric sample raw data may be reconstructed. In case of reconstruction instructions being required, the respective instructions may be used.

In another aspect of the present disclosure, at least one of the fragments created from the biometric sample raw data is an essential fragment. Essential fragments are those fragments which contain information of the biometric sample that is un-substitutable or un-rebuildable on the basis of the information of the respective other fragments. According to this, the biometric sample data cannot be reconstructed without the essential data. Fragmentation with creation of at least one essential fragment may include additional pre-processing, which would further have to be reversed to rebuild the raw data. Non-limiting examples for fragmentations with at least one essential fragment may be for images, e.g. face recognition, the detection of certain elements, e.g. the irises in the face, and to cut out a portion of the image including a essential element, e.g. a circular area such that it covers the whole eye and save this portion as an essential fragment, and divide the remainder in blocks NxN pixels in size, where N is number of entities storing this image. Another example includes the application of well known algorithms to detect main features of the image, e.g. a face (eyes, nose, lips, etc), randomly select areas of the image and apply effects/filters such as blur, noise to transform the image so the quality of the image is not good enough for biometrics but it might be sufficient to recognize the person by a human, store applied transformations locally in the terminal or in the electronic configuration file and partition the image into N pieces as previously described. Another example, e.g. applicable for voice recognition may be the application of a inverse Fourier transformation to detect key frequencies/cepstral coefficients, taking most impacting coefficient and mark it as the essential fragment, splitting remaining coefficients amongst other entities involved in the process.

In addition or alternatively to this, fragmentation may be done in such a way that reconstruction of the raw data is impossible without the respective reconstruction instructions. For example, in case of erratic fragmentations, the reconstruction instructions may be required for reconstructions of the raw data. In these cases, where the fragments are similar but the reconstruction instructions are essential for reconstructing the data, the reconstruction instruction file may be considered as equivalent to en essential fragment as described above.

In order to revert to the original image the user would have to reveal the applied transformations, the order they were applied and parts of the image that they were applied to.

In yet another aspect of the present disclosure, even though the essential fragment may be stored anywhere, the essential fragment is stored under the responsibility of the user. According to this, the essential fragment may be stored after fragmentation in, preferably, a secure storage on the terminal. If the fragmentation was performed on the server, the essential fragment may be transmitted to the terminal, preferably making use of secure communication channels, for subsequent storage.

In another aspect of the present disclosure, the fragments are consolidated upon request from the user and the raw data of the biometric sample is rebuilt from the fragments. In case reconstruction instructions are required, these may be consolidated together with the fragments. The request from the user may require additional authorisation for processing.

Depending on the type of biometric sample, but also on further requirements, doubtful authentication data may be compared directly with the raw data of the biometric sample. Alternatively or in addition, the raw data of the biometric sample may be processed, e.g. features may be extracted and a new template may be created, and the processed data may be used for comparison and verification.

In yet another aspect of the present disclosure, the consolidated data and the reconstructed data are deleted after re-extraction of the template and/or after finishing the verification process.

The present disclosure is also directed to a system for biometric template extraction and secure biometric sample raw data storage, the system comprising a terminal and at least one storage entity, wherein
the terminal comprises a scanning device for enrolling at least one biometric sample of a user and is executing an extraction application, a raw sample splitting application, and a fragment distribution application,
wherein the terminal is configured to enrol, by the scanning device, at least one biometric sample of the user, to extract, by the extracting application, a biometric template from the enrolled raw biometric data of the sample, to fragment, by the raw sample splitting application, the raw data of the biometric sample and to distribute, by the fragment distribution application, the fragments for storage on the terminal and the at least one storage entity.

The present disclosure is further directed to a terminal for performing the method of biometric template extraction and data fragmentation, the terminal comprising:
a scanning unit for capturing raw data of at least one biometric sample of a user;
a processing unit, configured to enrol at least one biometric sample of a user from the captured raw data, to extract a biometric template from the raw data, and to fragment the raw data; and
a communication unit, configured to transmit at least one of the created fragments to at least one storage entity.

In yet another aspect of the present disclosure, the terminal further comprises a storage unit for storing raw data of biometric sample and/or a fragment created from the raw data of the biometric sample. The storage unit may be part of a memory unit of the terminal or may be a separate memory unit. In a preferred embodiment, the storage unit is a secure storage unit.

The present disclosure is also directed to a system for biometric template extraction and secure biometric sample raw data storage, the system comprising a terminal, a server and at least one storage entity, wherein
a terminal comprising a scanning unit for capturing raw data of at least one biometric sample of a user and communication means for transmitting the captured raw data; a server comprising communication means to receive the raw data from the terminal, the server being configured to execute an extraction application to extract a biometric template from the raw data and a splitter application to fragment the raw data;
at least one independent entity comprising communication means to receive at least one fragment of the raw data from the server and storage means to store the received fragment.

Raw data of a biometric sample is captured by the scanning device of the terminal and is transmitted by the communication means of the terminal to the server. The raw data of the biometric sample is associated to user related data used for identifying the biometric raw data and to associate the biometric raw data to a user or a user profile during a subsequent enrolment process on the server. During the enrolment process a biometric template is extracted from the raw data of the biometric sample. The biometric template comprises key features of the biometric sample used in subsequent biometric identification processes. After biometric template extraction, a splitter application in executed on the server for fragmenting the raw data of the biometric sample as described above, which are then distributed to at least one storage entity and to the terminal.

The present disclosure is also directed to a computer program computer program product comprising program instructions for carrying out each of the method steps of the disclosure, when said product is executed on a computer.

Further, the present disclosure is directed to a computer readable medium storing program instructions, which, when executed by a processor of a computer cause the computer to perform each of the method steps of the disclosure.

One advantage that may be realized in the practice of some embodiments of the described methods is that raw data of biometric samples can electronically be stored securely and may be consolidated and rebuilt for verification of an inconclusive biometric authentication process. Other technical advantages of the present disclosure will be readily apparent to one skilled in the art from the following description of preferred embodiments and the claims. Various embodiments of the present application obtain only a subset of the advantages set forth. No single advantage is critical to the embodiments. Any claimed embodiment may be technically combined with any other claimed embodiments.

### Brief description of the Drawings

Fig.1 shows a flowchart biometric template extraction and the secure raw data storage process;
Fig. 2. shows a block diagram showing the entities involved in combination with the steps of the extraction, secure storage and consolidation process;
Fig. 3 shows an example of fragmentation including an essential fragment; and
Fig. 4 shows a block diagram showing fragmentation including an essential fragment.

The accompanying drawings illustrate exemplary embodiments of the disclosure and serve to explain, by way of example, the principles of the disclosure.

### Detailed description

The present disclosure will now be described more fully hereinafter with reference to the accompanying figures, in which preferred embodiments are shown, by way of example only. The method, however, may be embodied in many different forms and should not be construed as limited to the embodiments set forth herein. It should be noted that these figures are intended to illustrate the general characteristics of the methods utilized in certain embodiments. However, the figures may not precisely reflect the precise structure or performance characteristic of any given embodiment. Moreover, in the figures like reference numerals designate corresponding parts throughout the different views or embodiments.

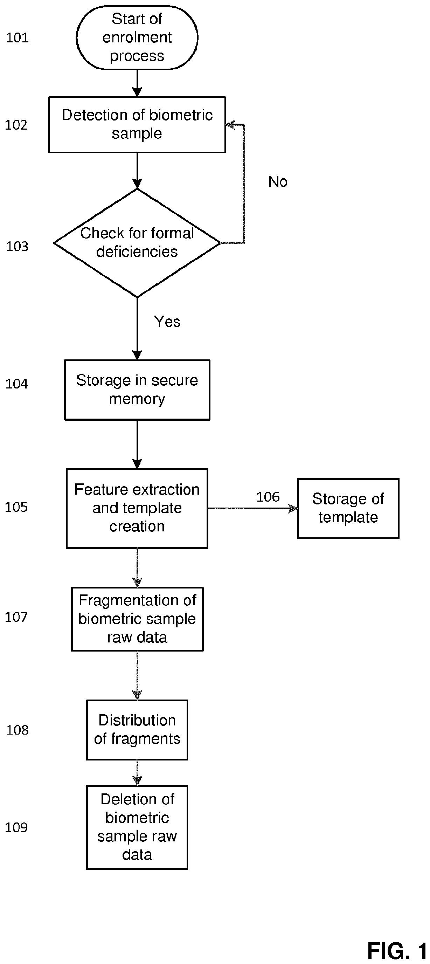

Fig. 1 is a flowchart illustrating exemplary the overall steps for biometric template extraction and secure biometric sample raw data storage. In step 101 the user starts the enrolment process for using the biometric authentication system by starting a dedicated application, namely e.g. a biometric authentication application, on his terminal, in this case a smartphone. The smartphone is equipped means for detection of the biometric sample, and detects, at step 102 a biometric sample. In this case a picture of the user's face is captured by the camera of smartphone. First, at step 103, the captured image is checked with regard to formal aspects, e.g. face position, exposure, focus, and the like, and, if the image fulfils the requirements for biometrics, it is approved and the enrolment process is continued.

At step 104, captured and approved image is stored in a secure portion of the memory of the smart phone. At step 105, an extraction application integrated into the biometric authentication application extracts the adequate features for the face recognition method at the required security level and extracts a template for use in biometric authentication. The template is stored (step 106) for future use, preferably in a secure portion of the memory.

At step 107 a raw sample splitter integrated in the biometric authentication application fragments the stored raw data of the biometric sample based security requirements. If fragments of the data are to be sent to three entities, e.g. third party trustees, four fragments are created, three for the entities, one to be kept on the user terminal. If a higher security level is selected, an essential fragment is created, in certain embodiments this essential fragment may be subject to stronger encryption that the remaining fragments. In the present example, the eye portion of the captured image of the face is cut out and stored, as the essential fragment on the user terminal. The remainder of the image is divided into three fragments. At the same time the biometric authentication application created an electronic configuration file for storing information regarding the fragmentation as well as the destinations of the fragments.

At step 108 the created fragments are sent to destination entities for storage. The destination entities, responsive to receiving the fragment, are sending a confirmation receipt to the terminal. The confirmation receipt is added to the electronic configuration file. Subsequently, at step 109, the raw data of the biometric sample is deleted from the memory of the smartphone.

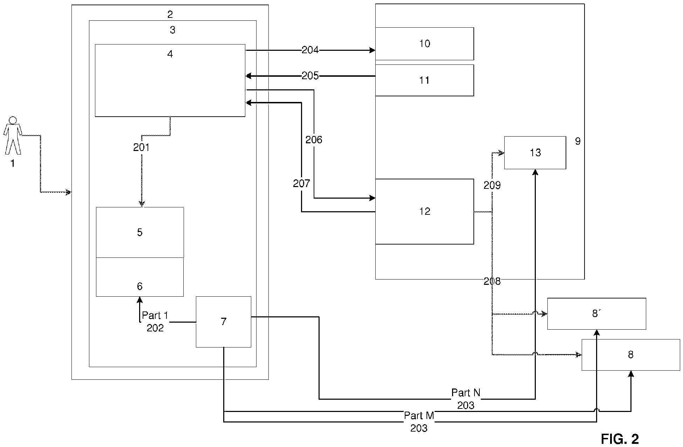

Fig. 2. shows a block diagram showing the entities involved in combination with the steps of the extraction, secure storage and consolidation process. A user 1 of a mobile device 2 makes use of biometric authentication. A biometric authentication application 3, interacting with the hardware of the mobile device and providing a user interface 4 is installed and executed on the mobile device. The biometric authentication application 3 includes an extraction application 5, a secure memory 6 and a raw sample splitter 7. When an image to be used for the biometric authentication system is captured, either for enrolment or for subsequent use for authentication purposes, the image is transferred 201 to the extraction application 5 for extraction of characteristic features and the creation of a biometric template. The picture used for enrolment is stored in the secure memory 6. Subsequently, the raw data of the biometric template is further processed by the raw sample splitter, wherein an essential fragment Part 1 and further fragments Part N, Part M are created. The essential fragment Part 1 is stored 202 in the secure memory 6 and the other parts Part N, Part M are distributed 203 to third party trustees 8, 8'and the storage system 13 service provider 9 for the biometric authentication services. When using the biometric authentication system, the biometric authentication application 3 on the mobile device 2, interacts and communicates 204, 205 with an enrolment portal 10 and a verification portal 11 in the responsibility of the service provider 9. In case of a complaint regarding a doubtful authentication, the user initiates a complaint request, which is submitted to the complaints portal 12 of the service provider. In order to deal with the complaint, the fragments are consolidated 207,209, 209 and the raw data is rebuilt.

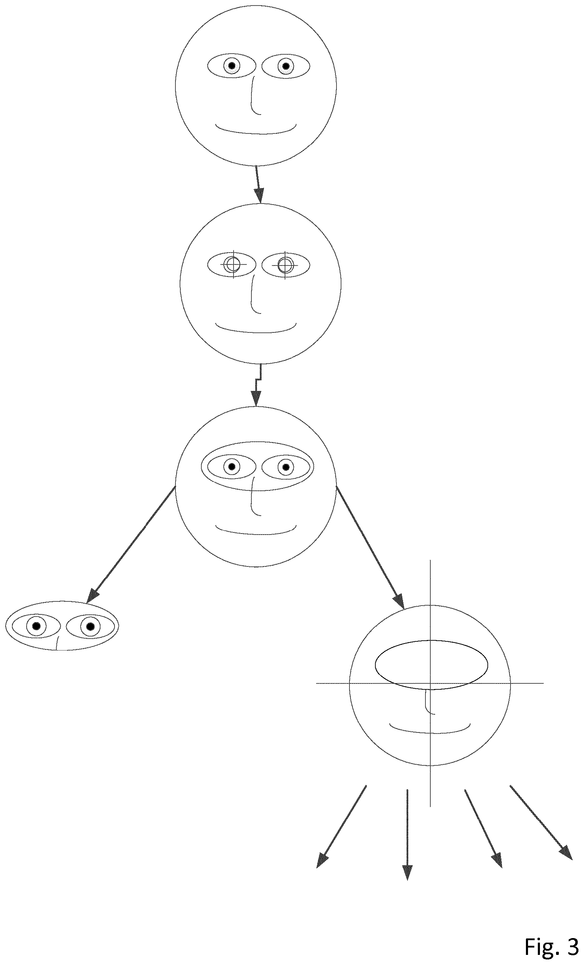

Fig. 3 shows an example of fragmentation including an essential fragment based on an image used for face recognition. In a face the irises are detected and circular areas covering the eyes and a surrounding portion of the eyes are marked. The necessary size of the marked area can be determined based on calculating the scale between the size of the iris and the size of an image pixel. Subsequently, the two marked areas are connected, resulting in an ellipsoidal shaped area. The ellipsoidal shaped area is then cut out and saved as an essential fragment. The remainder of the image is than divided into four fragments for subsequent distribution.

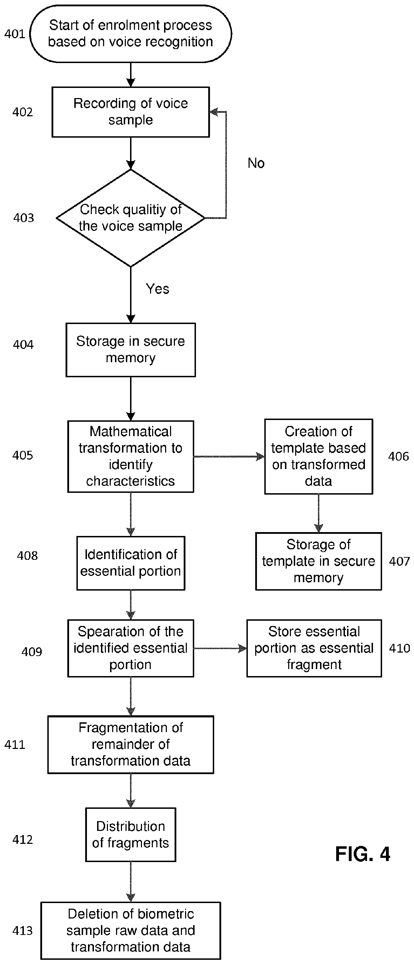

Fig. 4 shows a block diagram of the fragmentation process including the generation of an essential fragment based in the context of voice recognition. At a first step (step 401), the user starts the enrolment process based on voice recognition as biometric characteristic to be used. The user is prompted by the biometric authentication application on his terminal, in this case a smartphone, to speak into the detection means, i.e. the microphone of the smartphone in order to record at step 402 a voice sample. Subsequently, at step 403, the quality of the recorded voice sample is checked. If the quality of the recorded voice sample does not allow further processing, the step is repeated until a sample with sufficient quality has been recorded. If the voice sample fulfils the quality requirements, the sample is stored at step 404 in a secure portion of the memory of the smart phone.

At step 405 the recorded voice sample undergoes mathematical transformations in order to identify the voice characteristics to be used for biometric authentication. For this purpose the excitation signal produced by the glottis of the user is separated from the filter caused by the physical characteristics of the user. Known techniques are linear prediction and Fourier transformation based cepstral analysis, which allow detection of key frequencies and/or cepstral coefficients which are used for voice based biometric authentication. According to this, the filter data revealed by the mathematical transformation is used as the basis for the biometric authentication and the template used for biometric authentication is based on said filter data.

At step 406, a template for use in biometric authentication is extracted from the transformed data and, at step 407, stored in a secure portion of the memory of the smart phone.

At step 408, the most impacting characteristics of the filter revealed by the mathematical transformation is identified as an essential portion of the transformed data. At step 409, the essential portion of the data is separated and is stored (step 410) as essential data in a secure portion of the memory. In some embodiments the essential portion of the data has a stronger level of encryption than the remaining portions of the data.

At step 410 the remainder of the filter data is fragmented and, at step 412 distributed to the respective entities for storage. Subsequently, the raw data of the biometric sample, i.e. the voice recording, and the results of the mathematical transformation, i.e. the excitation signal and the filter data are deleted from the memory.

This description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art.

## Claims

1. A method for biometric template extraction and secure biometric sample raw data storage, the method comprising:
a) enrolling, at a terminal, at least one biometric sample of a user;
b) storing, in a secure storage on the terminal, the raw data of the biometric sample;
c) extracting, by an extraction application executed on the terminal, a biometric template from the raw data of the sample;
d) fragmenting, by a raw sample splitter executed on the terminal, the raw data of the biometric sample;
e) distributing the fragments of the raw data of the biometric samples from the terminal to independent entities, preferably via a secure communication channel; and
f) deleting the biometric sample raw data from the secure storage of the terminal.

2. A method for biometric template extraction and secure biometric sample raw data storage, the method comprising:
a) enrolling, at a terminal, at least one biometric sample of a user;
b) transmitting, preferably via a secure communication channel, the raw data of the biometric sample from the terminal to a server;
c) extracting, by an extraction application executed on the server, a biometric template from the raw data of the sample;
d) fragmenting, by a raw sample splitter executed on the server, the raw data of the biometric sample;
e) distributing the fragments of the raw data of the biometric samples from the server to independent entities, preferably via a secure communication channel; and
f) deleting the biometric sample raw data from the memory of the server.

3. The method according to claim 1 or 2, wherein independent entity is selected from the group consisting of physically separated entities, electronically separated entities and/or distinct and separate legal entites and/or combinations thereof.

4. The method according to any of the preceding claims, wherein the fragmenting process produces at least two fragments.

5. The method according to claim 4, wherein the number of fragments corresponds to the number of entities used for storing the fragments.

6. The method according to claims 4 or 5, wherein at least one of the at least two fragments is an essential fragment which contains information of the biometric sample that is un-substitutable or un-rebuildable on the basis of the information of the respective other fragments, and, optionally,
wherein the essential fragment is stored in a secure storage on the terminal.

7. The method according to any of the preceding claims, wherein the fragments consolidated upon a request from the user and the raw data of the biometric sample is rebuild from the fragments.

8. The method according to claim 7, wherein the biometric template is extracted from the rebuilt biometric sample.

9. The method according to claim 8, wherein the data used for biometric authentication or identification is compared with the extracted biometric template.

10. The method according to claim 7, wherein the consolidated fragments and the rebuilt biometric template are deleted after extraction of the biometric template.

11. A system for biometric template extraction and secure biometric sample raw data storage, the system comprising a terminal and at least one storage entity, wherein
the terminal comprises a scanning device for enrolling at least one biometric sample of a user and is executing an extraction application, a raw sample splitting application, and a fragment distribution application,
wherein the terminal is configured to enrol, by the scanning device, at least one biometric sample of the user, to extract, by the extracting application, a biometric template from the enrolled raw biometric data of the sample, to fragment, by the raw sample splitting application, the raw data of the biometric sample and to distribute, by the fragment distribution application, the fragments for storage on the terminal and the at least one storage entity.

12. A terminal for performing the method according to claim 1, the terminal comprising:
a capturing unit for capturing information related to a biometric sample of a user;
a processing unit, configured to enrol at least one biometric sample of a user from the captured information, to extract a biometric template from the biometric sample, and to fragment the biometric template; and
a communication unit, configured to transmit at least part of the created fragments of the biometric template to independent entities.

13. A system for biometric template extraction and secure biometric sample raw data storage, the system comprising
a terminal comprising capturing means for capturing information related to a biometric sample of a user and communication means for transmitting the captured raw data of the biometric sample;
a server comprising communication means to receive the raw data from the terminal, the server being configured to execute an extraction application to extract a biometric template from the raw data and a splitter application to fragment the raw data;
at least one independent entity comprising communication means to receive at least one fragment of the raw data from the server and storage means to store the received fragment.

14. A computer program product comprising computer executable instructions embodied in a computer readable medium for performing steps any of the method claims 1 to 10.

15. A computer readable medium storing program instructions, which, when executed by a processor of a computer cause the computer to perform each of the method steps of claims 1 to 10.
